# EUROPEAN PATENT APPLICATION

(11) **EP 3 578 174 A1**
(43) Date of publication of application: **11.12.2019**
(21) Application number: 17916830.7
(22) Date of filing: 07.07.2017
(51) Int. Cl.: A61K 31/045, A61P 25/28

(54) **USE OF (+)-2-BORNEOL IN PREPARATION OF DRUG FOR PROMOTING UPREGULATION OF EXPRESSION OF SPHINGOSINE KINASE-1 AND/OR BDNF**

(71) Applicant: Suzhou Pharmavan Cancer Research Center Co., Ltd., Suzhou, Jiangsu 215000 (CN)
(72) Inventor: LI, Yunsen, Suzhou Jiangsu 215000 (CN); DENG, Shiping, Suzhou Jiangsu 215000 (CN); LI, Yong, Suzhou Jiangsu 215000 (CN); JIANG, Chuanliang, Suzhou Jiangsu 215000 (CN); YU, Yunhui, Suzhou Jiangsu 215000 (CN)
(74) Representative: Plougmann Vingtoft a/s
(86) International application number: PCT/CN2017/092148
(87) International publication number: WO 2019/006734

(57) **Abstract**

Disclosed is the use of (+)-2-borneol in the preparation of a drug for promoting the upregulation of the expression of sphingosine kinase-1 and/or BDNF (a brain-derived neurotrophic factor). Herein, (+)-2-borneol can be used to prepare a drug for promoting the upregulation of the expression of the sphingosine kinase-1 and/or the brain-derived neurotrophic factor. The drug can induce astrocyte spreading and migration, oligodendrocyte differentiation and survival, and neurite growth and nerve regeneration, and can promote the upregulation of the expression of the brain-derived neurotrophic factor, promote the survival of neurons and the growth of axons, inhibit the expansion of the infarct volume, and achieve the effect of repairing damage at the site of an immediate injury while preventing further expansion of the infarct area to completely treat brain damage, so that the long-term therapeutic effect thereof is significantly improved.

## Description

### Technical Field

The present application belongs to the field of chemical medicine and relates to use of (+)-2-borneol in the preparation of a drug for promoting up-regulation of sphingosine kinase-1 and/or BDNF (brain-derived neurotrophic factor) expressions.

### Background

Neuronal ischemia, inflammation, immune response, trauma, neuronal degeneration and other reasons can cause neuronal damage or death, resulting in serious neurological or psychiatric diseases. Therefore, neuroprotective therapeutic drugs that can delay nerve damage or death or promote nerve cell growth are particularly important for life and health.

Neuroprotection is focused on protecting, recovering, healing, or regenerating the structure or function of cells of the nervous system (J Clin Neurosci. 2002 Jan; 9(1):4-8). The purpose of neuroprotection is to prevent or minimize initial damages to the nervous system, or to prevent or minimize endogenous or exogenous harmful processes that cause damage to axons, neurons, synapses, and dendrites.

Neuroprotective agents are currently in different research and experimental stages and are available in a wide variety, including the following types: ion channel modulators, excitatory amino acid antagonists [N-methyl-D-aspartate (NMDA) antagonists, α-amino-3-hydroxy-5-methyl-4-isoxazolepropionic acid (AMPA) antagonists], nitric oxide synthase (NOS) inhibitors, free-radical trapping agents/scavengers, magnesium sulfate, GABA receptor enhancers, anti-inflammatory agents, and cell membrane stabilizers, *etc.* ("Chinese guidelines for diagnosis and treatment of acute ischemic stroke 2014"; Neuropharmacology, 2008, 55(3): 363-389).

However, the ability of these drugs in research to limit secondary biochemical damage and cell death is disappointing (Arch Neurol. 2007 Jun; 64(6): 794-800). Currently, there are no clinically evidence-based neuroprotective drugs on the market ("Chinese guidelines for diagnosis and treatment of acute ischemic stroke 2014").

Neurotrophins are growth factors that regulate the development and maintenance of the peripheral and central nervous systems (Annu Rev Neurosci. 1996; 19: 289-317). Nerve growth factor (NGF) is the first discovered and best characterized member of the neurotrophin family. Other structurally related proteins, such as brain-derived neurotrophic factor (BDNF), are included in this family. Neurotrophins act through two major signaling pathways: the phosphatidylinositol-3-kinase (PI3K)-AKT pathway and the mitogen-activated protein kinase (MAPK)-MEK pathway, both of which involve inhibition of apoptosis. It is also known that neurotrophins can act on mature neurons and, in particular, on damaged and denatured cells (Curr Neurovasc Res. 2007 May; 4(2): 143-51).

It has been confirmed by several researchers that NGF is a potential therapeutic agent for several diseases. Such diseases include stroke, neurodegenerative diseases, neuroinflammation, and some types of cancers, multiple sclerosis, *etc.* (Curr Alzheimer Res. 2007 Dec; 4(5): 503-6; Curr Alzheimer Res. 2008 Feb; 5(1): 38 -44). NGF has significant immunomodulatory properties during CNS (central nervous system) inflammation to promote maintenance of CNS properties (Prog Brain Res. 2004; 146: 403-14). In addition to its neuroprotective properties in neurons and oligodendrocytes, NGF also induces immunosuppression during autoimmune demyelination. This finding makes it an excellent candidate for the treatment of CNS inflammatory diseases such as MS. However, NGF is not an ideal drug candidate due to its inability to cross the blood-brain barrier, its short half-life, and its side effects.

For many years, researchers have been finding small molecule drugs that have NGF agonist activity or can up-regulate NGF expression, and have better pharmacokinetic properties and fewer side effects.

In addition, sphingolipids are one of the important structural components of cell membranes. Their metabolites, such as ceramide, sphingosine, and sphingosine 1-phosphase (SIP), are also biologically active signaling molecules and can act as first and/or second messengers for regulating the life activities of cells, such as cell survival, proliferation, migration, and neovascularization, *etc.* Sphingosine kinase is a rate-limiting enzyme that regulates the balance between ceramide and SIP. In recent years, studies have shown that ceramide, sphingosine kinase, and S1P may be involved in multiple processes in the process of cerebral ischemia, suggesting that intervention of this pathway can be used as a new target for the treatment of cerebral ischemia. SIP and the like are directly involved in the regulation of cell proliferation and apoptosis, and can promote various biological functions such as cell growth, proliferation, and anti-inflammation. The cell membrane receptor of SIP is a G protein-coupled receptor. Upon binding of SIP and the receptor thereof, different signaling pathways are activated, and thereby cell functions can be regulated in different ways. Sphingosine kinase is an important rate-limiting enzyme that maintains the balance between ceramide, sphingosine, and SIP in cells, and is also an important signaling molecule that affects cell survival and proliferation.

SIP (sphingosine 1-phosphate) is a biologically active sphingolipid metabolite, and brain is the organ with the highest concentration of SIP (Nat Rev Immunol 2005, 5: 560-570). SIP receptors are widely expressed in neurons, astrocytes, and microglia (Pharmacol. Ther. 2008, 117: 77-93). Asegawa et al. (Biochim Biophys Acta. 2002; 1585: 193-201) have found that S1P plays a protective effect on cerebral ischemia by activating S1P1 in a rat model of cerebral ischemia. This effect is associated with the activation of Akt (protein kinase B) which plays an important role in the inhibition of apoptosis. In a related study on the inhibition of apoptosis by SIP (Biochim Biophys Acta. 2008, 1781: 459-466), it is believed that Akt inhibits apoptosis through the following mechanism: SIP is coupled with sphingosine kinase receptor to activate Akt, which in turn blocks the release of cytochrome C induced by Bad (an apoptotic precursor protein), thereby inhibiting apoptosis. Moreover, SIP can also induce the proliferation of neuronal progenitor cells (J Neurochem. 2007, 103: 509-517), enhance the tolerance of cortical neurons and endothelial cells to ischemia and hypoxia, and counteract brain cell death caused by ischemia and hypoxia. Since SIP can prevent apoptosis, it can be used to treat diseases caused by cerebral ischemia, *etc.,* providing a new way for us to find new drugs for treating stroke.

Sphingosine kinase (SphK), a key enzyme that catalyzes production of SIP from sphingosine, is essential for the regulation of SIP levels. Sphingosine kinase 1 (Sphkl) and sphingosine kinase 2 (Sphk2) are key enzymes that promote the metabolism of sphingomyelin into S1P (Stroke, 2011, 42: 1420-1428). An increasing number of studies have shown that the Sphk1/S1P signaling pathway can regulate neurotransmitter release, neuroinflammation, and proliferation and death of neurons and microglia [J Clin Invest, 2009, 119(7): 1871- 1879]. Orhan Altay et al. (FASEB J, 2011, 25(2): 600-612) have also found that Sphk1/SIP also regulates the permeability of the blood-brain barrier following subarachnoid hemorrhage. Frank Niessen's study (Mol Cell Biol, 2005, 25(24): 11113-11121) has also shown that the SphKs/S 1P pathway is an important molecular mechanism that promotes nerve growth and inhibits apoptosis of dendritic cells. Therefore, Sphkl may also be an important protein that we have not previously recognized and can regulate pathological damage such as cerebral ischemia, and small molecules that can up-regulate Sphkl expression are also potential drugs for treating nerve cell injury diseases.

Therefore, in this field, it is desired to develop a new drug for treating nerve cell injury diseases.

### Summary of the Invention

In view of the deficiencies in the prior art, the object of the present application is to provide use of (+)-2-borneol in the preparation of a drug for promoting up-regulation of sphingosine kinase-1 and BDNF (brain-derived neurotrophic factor) expressions.

To achieve the object, the present application adopts the following technical solutions:
In one aspect, the present application provides use of (+)-2-borneol in the preparation of a drug for promoting up-regulation of sphingosine kinase-1 and/or BDNF (brain-derived neurotrophic factor) expressions.

The (+)-2-borneol as described in the present application can be used in the preparation of a drug for promoting up-regulation of sphingosine kinase-1 and brain-derived neurotrophic factor expressions. This drug can induce astrocyte proliferation and migration, oligodendrocyte differentiation and survival, neurite growth, and nerve regeneration, and can promote the up-regulation of brain-derived neurotrophic factor expression, promote neuron survival and axon growth, and inhibit the expansion of infarct volume. Therefore, the drug as described in the present application can achieve a damage-repairing effect on the directly-damaged site while preventing further expansion of the infarct area.

In the present application, the (+)-2-borneol has a chemical formula of C₁₀H₁₈O and a molecular weight of 154.25, and has the structural formula as shown in Formula I:

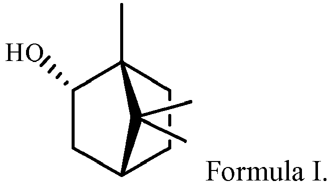

Preferably, the expression of sphingosine kinase-1 is up-regulated by 2-4 fold, e.g., 2 fold, 2.3 fold, 2.5 fold, 2.7 fold, 2.9 fold, 3 fold, 3.2 fold, 3.4 fold, 3.6 fold, 3.8 fold, or 4 fold under the action of the drug for promoting up-regulation of sphingosine kinase-1 and/or brain-derived neurotrophic factor expressions.

Preferably, the expression of brain-derived neurotrophic factor is up-regulated by 2-4 fold, e.g., 2 fold, 2.3 fold, 2.5 fold, 2.7 fold, 2.9 fold, 3 fold, 3.2 fold, 3.4 fold, 3.6 fold, 3.8 fold, or 4 fold under the action of the drug for promoting up-regulation of sphingosine kinase-1 and/or brain-derived neurotrophic factor expressions.

In the present application, the drug for promoting up-regulation of sphingosine kinase-1 and/or brain-derived neurotrophic factor expressions contains (+)-2-borneol as an active ingredient, but does not contain other active ingredients.

Preferably, the drug simultaneously promotes up-regulation of sphingosine kinase-1 and/or brain-derived neurotrophic factor expressions. The simultaneous up-regulation of the both expressions can prevent further expansion of the cerebral infarct area and repair the damaged site so as to completely recover the brain damage, allowing significant improvements in long-term treatment effects.

Preferably, the drug further comprises a pharmaceutically acceptable carrier. The pharmaceutically acceptable carrier can be selected according to techniques well known in the art and is not particularly limited in the present application.

Preferably, the drug further comprises an excipient. The excipient can be selected by those skilled in the art according to actual needs and is not particularly limited in the present application.

Preferably, the drug is in a dosage form of capsule, tablet, granule, powder, injection, or dropping, and preferably injection.

The drug for promoting up-regulation of sphingosine kinase-1 and/or brain-derived neurotrophic factor expressions as described in the present application can be used for the treatment of cranial nerve injury diseases such as stroke and Alzheimer's disease, *etc.*

Compared with the prior art, the present application has the following beneficial effects:
In the present application, (+)-2-borneol can be used in the preparation of a drug for promoting up-regulation of sphingosine kinase-1 and/or brain-derived neurotrophic factor expressions. The drug can induce astrocyte proliferation and migration, oligodendrocyte differentiation and survival, and neurite growth and nerve regeneration, and can promote up-regulation of brain-derived neurotrophic factor expression, promote neuron survival and axon growth, and inhibit the expansion of infarct volume, achieving a damage-repairing effect on the directly-damaged site while preventing further expansion of the infarct area so as to completely recover the brain damage, allowing significant improvements in long-term treatment effects.

### Description of the Drawings

Figure 1 shows the expression of sphingosine kinase 1 for the administration group and the control group after administration of (+)-2-borneol to a rat model of focal cerebral ischemia-reperfusion;
Figure 2 shows the expression of brain-derived neurotrophic factor BDNF for the administration group and the control group after administration of (+)-2-borneol to a rat model of focal cerebral ischemia-reperfusion;
Figure 3 is a graph showing changes in failure rate of the left forelimb of mice in various experimental groups;
Figure 4 is a graph showing changes in failure rate of the right forelimb of mice in various experimental groups;
Figure 5 is a graph showing changes in the asymmetry index of mice in various experimental groups;
Figure 6 is a graph showing changes in body weight of mice in various experimental groups;
Figure 7 is a graph showing the measurement results of the dendritic length around the ischemic region of mice in various experimental groups;
Figure 8 is a graph showing the measurement results of the number of dendritic branches around the ischemic region of mice in various experimental groups;
Figure 9 is a graph showing the morphology changes of the dendritic branches around the ischemic region of mice in various experimental groups;
Figure 10 is a graph showing the measurement results of dendritic spine density around the ischemic region of mice in various experimental groups;
wherein, *P<0.05, compared with the model group.

### Detailed Description

The technical solutions of the present application are further described by reference to specific embodiments below. It should be understood by those skilled in the art that the examples are provided only for the purpose of understanding the present application and should not to be considered as limiting the scope of the present application.

### Example 1

### Rat model of focal cerebral ischemia-reperfusion

A rat model of middle cerebral artery occlusion (MCAO)-induced cerebral ischemia-reperfusion was prepared by thread occlusion of the internal carotid artery. A drug was administered once by tail vein injection 2 hours after ischemia-reperfusion. One dose group was set for (+)-2-borneol, *i.e.* 2 mg/kg. 48 hours after the cerebral ischemia-reperfusion, about a soybean size of brain tissue around the infarct area was harvested, sent to CapitalBio Technology Inc., Beijing, and subjected to genome-wide expression profiling by using Affymetrix GeneChip Rat Genome 230 2.0 Array Affymetrix Rat (Latin: *Rattus norvegicus*) Genome 230 2.0 chip.

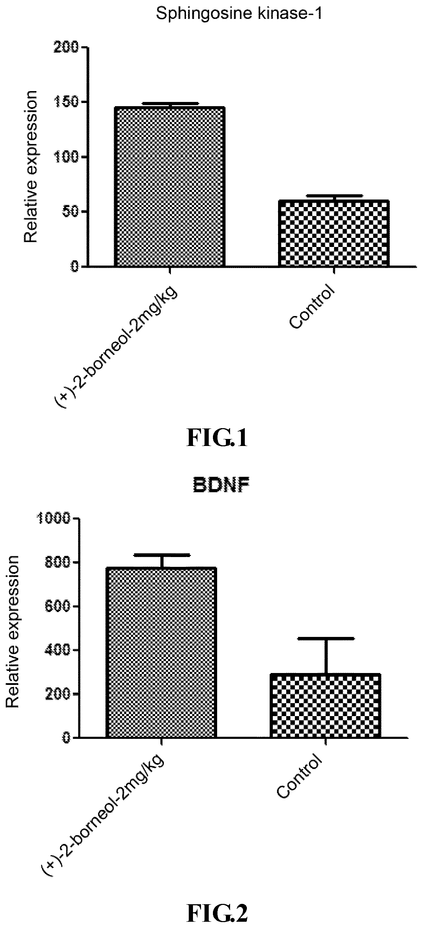

The results showed that: relative to the sham-operated group, the (+)-2-borneol administration group showed that:
(1) the sphingosine kinase-1 (Sphkl) expression was up-regulated by 2.64 fold (as shown in Figure 1). Sphkl can phosphorylate sphingosine to produce SIP which acts directly on brain cells to induce astrocyte proliferation and migration, oligodendrocyte differentiation and survival, neurite growth, and nerve regeneration. (+)-2-borneol was administered to a rat model of MACO 2 hours after its establishment, and then it was foundthat Sphkl for the administration group was up-regulated by 2.64 fold relative to the model group (as shown in Figure 1), revealing that (+)-2-borneol may have a role in promoting neurite growth and nerve cell regeneration.
(2) the brain-derived neurotrophic factor (BDNF) expression was up-regulated by 2.67 fold (as shown in Figure 2). The up-regulation of BDNF can promote neuronal survival and axon growth, and inhibit expansion of the infarct volume. The neuroprotective effects of BDNF may be related to new gene transcription, protein synthesis, and regulation of protein kinases.

### Example 2

### Long-term pharmacodynamic assay of (+)-2-borneol in a mouse model of focal motor cortex ischemia

A mouse model of focal cerebral motor cortex ischemia was induced by photochemical method. Three dose groups were set for (+)-2-borneol, i.e. 3.0, 1.5, 0.75 mg/kg, respectively. Edaravone at a dose of 9 mg/kg was used as a positive control. A drug was administered once by tail vein injection 2 hours after cortex ischemia, and then administered every 24 hours, for a total of 14 administrations. A grid test was used to determine respectively the failure rate of forelimb on Day 14, Day 28 and Day 42 after ischemic injury; and a cylinder test was used to determine the motor asymmetry index of the affected forelimb and the contralateral forelimb on Day 14, Day 28 and Day 42 after ischemic injury to determine its effect on motor function. After the behavior measurements, 5 animals were randomly selected from each group and killed, and the brain was harvested for Golgi staining to determine the neuronal survival and dendritic richness in the penumbra area.

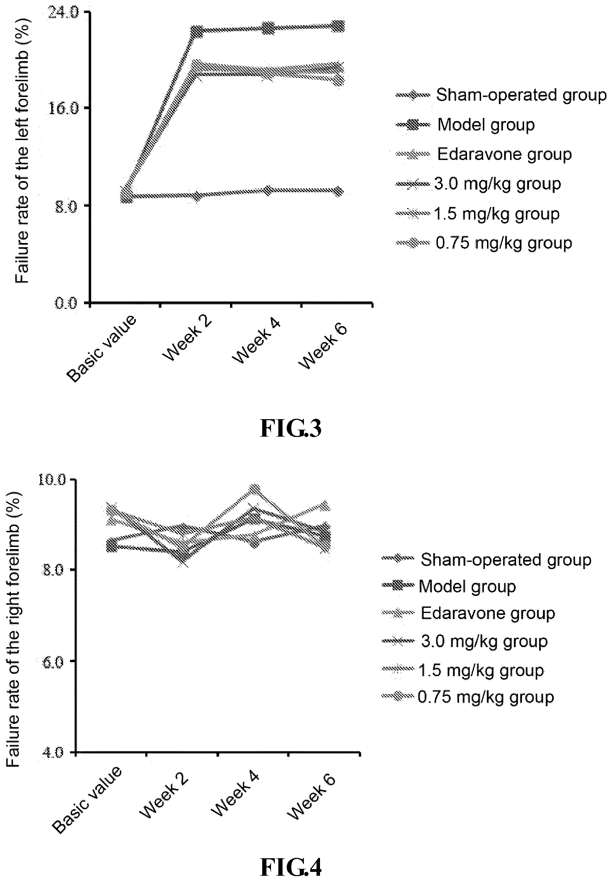

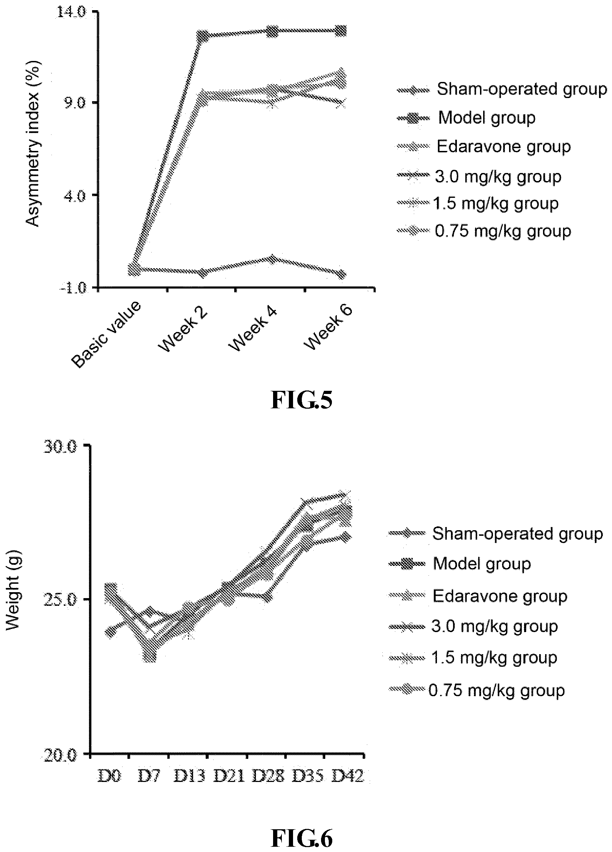

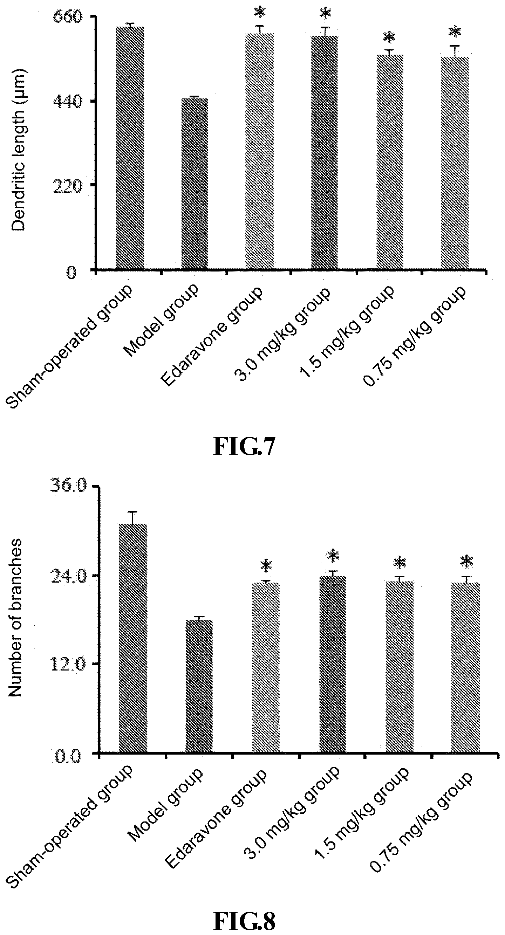

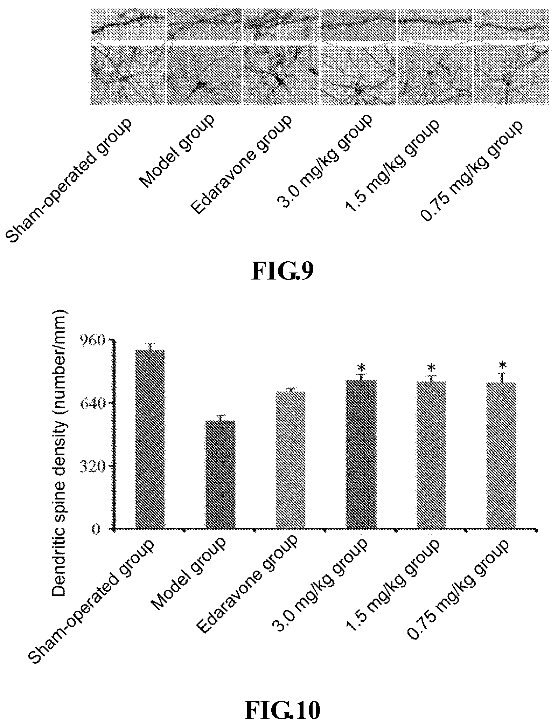

The results showed that the continuous, multiple administration of (+)-2-borneol after photochemically inducing the ischemic injury can significantly improve the motor function of mice with photochemically induced injury, comprising significant decreases in the failure rate of the left forelimb (affected side) and the asymmetry index of both forelimbs in mice (as shown in Figures 3 to 6), and significant improvements in the dendritic length (as shown in Figure 7), the number of dendritic branches (as shown in Figures 8 and 9), and the dendritic spine density (as shown in Figure 10) around the ischemic area.

Therefore, the (+)-2-borneol of the present application can promote up-regulation of sphingosine kinase-1 and/or BDNF (brain-derived neurotrophic factor) expressions, and it has a well long-term therapeutic effect.

The applicant states that use of (+)-2-borneol in the preparation of a drug for promoting up-regulation of sphingosine kinase-1 and BDNF expressions according to the present application has been demonstrated herein through the above examples, and however, the present application is not limited to the above examples, that is, it does not mean that implementation of the present application must rely on the above detailed examples. It should be apparent to those skilled in the art that, any improvement of the present application, the equivalent replacement of the materials used in the present application, the addition of auxiliary components, and the selection of specific modes, etc., will all fall within the protection scope and the disclosure scope of the present application.

## Claims

1. Use of (+)-2-borneol in the preparation of a drug for promoting up-regulation of sphingosine kinase-1 and/or brain-derived neurotrophic factor expressions, wherein the (+)-2-borneol has the structure of Formula I:

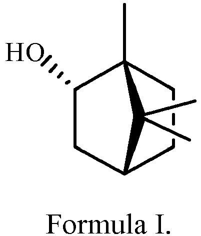

2. The use according to claim 1, wherein the expression of sphingosine kinase-1 is up-regulated by 2-4 fold under the action of the drug for promoting up-regulation of sphingosine kinase-1 and/or brain-derived neurotrophic factor expressions.

3. The use according to claim 1 or 2, wherein the expression of brain-derived neurotrophic factor is up-regulated by 2-4 fold under the action of the drug for promoting up-regulation of sphingosine kinase-1 and/or brain-derived neurotrophic factor expression.

4. The use according to any one of claims 1 to 3, wherein the drug simultaneously promotes the up-regulation of sphingosine kinase-1 and brain-derived neurotrophic factor expressions.

5. The use according to any one of claims 1 to 4, wherein the drug further comprises a pharmaceutically acceptable carrier.

6. The use according to any one of claims 1 to 5, wherein the drug further comprises an excipient.

7. The use according to any one of claims 1 to 6, wherein the drug is in a dosage form of capsule, tablet, granule, powder, injection, or dropping.

8. The use according to claim 7, wherein the drug is in a dosage form of injection.

9. Use of (+)-2-borneol in the preparation of a drug for treating a cranial nerve injury disease or condition, wherein the (+)-2-borneol has the structure of Formula I:

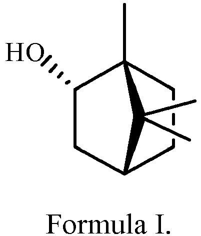

10. The use according to claim 9, wherein the cranial nerve injury disease or condition is caused by focal cerebral ischemia or focal cerebral ischemia-reperfusion; preferably, the cranial nerve injury disease or condition is stroke and/or Alzheimer's disease.

11. The use according to claim 9 or 10, wherein the drug further comprises a pharmaceutically acceptable carrier and/or an excipient.

12. The use according to any one of claims 9 to 11, wherein the drug is in a dosage form of capsule, tablet, granule, powder, injection, or dropping, and preferably injection.
